# EUROPEAN PATENT SPECIFICATION

(11) **EP 1 257 310 B1**
(45) Date of publication and mention of the grant of the patent: **15.06.2005**
(21) Application number: 01912842.0
(22) Date of filing: 20.02.2001
(51) Int. Cl.: A61M 5/315

(54) **SYRINGES AND SYRINGE SYSTEMS FOR SELECTIVELY DISPENSING CONTROLLED AMOUNTS OF A THERAPEUTIC SUBSTANCE**
SPRITZEN UND SPRITZENSYSTEME ZUR SELEKTIVEN VERABREICHUNG VON KONTROLLIERTEN MENGEN EINER THERAPEUTISCHEN SUBSTANZ
SERINGUES ET DISPOSITIFS DE SERINGUES POUR ADMINISTRER DE MANIERE SELECTIVE DES DOSES CONTROLEES D'UNE SUBSTANCE THERAPEUTIQUE

(30) Priority: 23.02.2000 US 511134
(43) Date of publication of application: 20.11.2002
(73) Proprietor: Hospira, Inc., Lake Forest, Illinois 60045 (US)
(72) Inventor: GRABENKORT, Richard, W., Barrington, IL 60010 (US); ZIEGLER, John, S., Arlington Heights, IL 60005 (US); SPRAGUE, Karl, J., Gurnee, IL 60031 (US)
(74) Representative: Modiano, Guido, Dr.-Ing.
(86) International application number: PCT/US2001/005375
(87) International publication number: WO 2001/062319

(56) References cited:
- EP-A- 0 363 338
- EP-A- 0 904 792
- EP-A- 0 911 046
- WO-A-01/00261
- WO-A-93/10842
- CA-A- 2 214 468
- US-A- 2 869 541
- US-A- 3 052 239
- US-A- 4 391 272
- US-A- 5 298 024
- US-A- 5 688 250
- US-A- 5 833 669

## Description

### TECHNICAL FIELD

This invention relates to syringes and syringe systems for packaging and delivering therapeutic substances or medicaments to a living body. The invention also relates to syringe systems for mixing a controlled amount of a first therapeutic substance or constituent, such as a liquid diluent with a second therapeutic substance or constituent, such as a dry or powdered drug, for subsequently dispensing the constituent mixture and for subsequently flushing leftover amounts of the second constituent from the syringe system with a residual supply of the first constituent.

### BACKGROUND OF THE INVENTION AND TECHNICAL PROBLEMS POSED BY THE PRIOR ART

It is known in the prior art to provide syringe systems that incorporate two pre-filled syringe barrels which cooperate to provide for the mixing of two constituents previously maintained in isolation from one another. Such systems are useful in pharmaceutical applications where pre-mixed solutions or suspensions of a drug are not stable enough to withstand prolonged periods of storage. Frequently, one of the two constituents is provided as a dry lyophilized, powdered drug and the other constituent is provided as a diluent. A first syringe barrel contains the dry drug within an expandable mixing chamber defined by a barrel wall, a dispensing end and a reciprocable stopper housed in the first syringe barrel. A second syringe barrel contains a supply of diluent for reconstituting the drug. Using a movable plunger reciprocably disposed in the second syringe barrel, diluent is displaced from the second syringe barrel through a discharge passage, through the stopper in the first syringe barrel and into the expandable mixing chamber where the dry drug is reconstituted. Once a predetermined supply of diluent has been discharged from the second syringe barrel, continued movement of the second syringe within the first syringe barrel results in dispensing of the mixed drug to a patient. These so-called "wet/dry" drug delivery systems are disclosed in U.S. Patent No. 5,779,668.

In many cases, the active drug in a two-constituent delivery system is substantially more expensive than the diluent. Thus, it is economically desirable to maximize the amount of active drug dispensed from the syringe system to the patient. In prior art systems, however, when the diluent supply has been exhausted from the diluent syringe, there often remains a residual amount of drug in the system. In particular, drug may remain in small amounts in the mixing chamber and in the syringe dispensing passage. As such, the amount of drug packaged in the pre-filled system is typically in excess of the actual amount needed to fulfill patient dosage requirements. This results in increased cost.

It would therefore be desirable to provide an improved syringe system that permits an increased amount of drug to be dispensed from the mixing chamber in comparison to prior art syringes and systems. In particular, it would be advantageous to provide an improved syringe which provides for the dispensing of a first predetermined amount of diluent or first constituent for mixing with a supply of drug or second constituent while providing for the retention of a residual supply of diluent or first constituent for flushing remaining amounts of drug from the mixing chamber.

It would also be beneficial to provide an improved syringe system which provides the aforementioned desirable features and which is economical to manufacture and use. Additionally, it would be desirable if such an improved syringe and syringe system could be easily and safely operated by an operator administering the therapeutic substance.

Patents EP-A-0904792, US-A-2,868,541, EP-A-0911046 and US-A-5,298,024 disclose syringes comprising a barrel having an open end an opposite delivery end defining a delivery passage, a plunger and a releasable stop operatively associated with the plunger and for defining an intermediate position between the retracted position and a final position in which the plunger is completely introduced in the barrel.

### SUMMARY OF THE INVENTION

The benefits and advantages described above are realized by the present invention which provides a syringe that permits a user to discharge a first predetermined amount of diluent, while reserving a residual supply of diluent for later use. A syringe according to the invention includes a barrel including a discharge end defining a discharge passage, a plunger disposed within the barrel, the plunger and discharge end defining a chamber within the barrel, the plunger being adapted to move within the barrel and thereby displace fluid from the barrel through a discharge end, and a releasable stop or limiting means that is operatively associated with the syringe plunger and adapted to move between a deployed position, in which the plunger movement is limited such that a first supply of fluid is displaced from the barrel while a residual supply of fluid remains in the barrel, and a retracted position, in which further movement of the plunger is permitted to displace the residual supply of fluid from the barrel.

Syringes according to the invention find particular application as diluent syringes in a wet/dry drug delivery syringe system having two cooperating syringe barrels. Accordingly, a prefilled syringe system according to the invention comprises a first barrel having an open end and an opposite mixture delivery end defining a mixture delivery passage; a stopper slidably disposed in the first barrel, the stopper and mixture delivery end defining a first chamber for containing the first constituent; a second barrel disposed within the first barrel, the second barrel having an open end and an opposite discharge end defining a discharge passage for conveying the second constituent into the first chamber of the first barrel; a plunger disposed within the second barrel, the plunger and discharge end defining a second chamber within the barrel, the plunger being adapted to move within the barrel and thereby displace the second constituent from the second barrel through the discharge end and into the fist chamber of the first barrel for mixing with the first constituent; and a releasable stop or limiting means that is operatively associated with the plunger and adapted to move between a deployed position, in which the plunger movement is limited such that a first supply of the second constituent is displaced from the second barrel while a residual supply of fluid remains in the second barrel, and a retracted position, in which further movement of the plunger is permitted to displace the residual supply of the second constituent from the second barrel.

The releasable stop or limiting means according to the present invention may take various forms. In one preferred embodiment, the releasable stop is provided as a thumb lever integrally molded with the syringe plunger and having an engaging end for engaging a portion of the syringe barrel when the thumb lever is in a deployed position. The thumb lever pivots relative to the plunger shaft about a pivot end opposite the engaging end and is biased in an outward radial direction from the longitudinal axis of the plunger shaft. When a lateral force is applied to a gripping surface of the thumb lever, the thumb lever pivots within a channel formed in the plunger shaft radially inward towards the plunger shaft axis to a retracted position. Preferably, the thumb lever includes a bi-stable, over-center detent which maintains the thumb lever in in the retracted position once pivoted beyond a center point. The plunger shaft is then permitted to continue to travel or move within the syringe barrel. The thumb lever may be actuated with the same hand used to actuate the plunger within the syringe barrel and therefore provides single-hand operation.

According to another preferred embodiment, the releasable stop is provided as a compressible cylindrical element situated around the plunger shaft beneath the plunger push button. In a deployed position, the cylindrical element assumes an external diameter that is larger than the internal diameter of the syringe barrel such that an engaging end of the cylindrical element prevents movement of the plunger. The cylindrical element may be compressed by application of a suitable lateral force to a retracted position in which the external diameter of the cylindrical element becomes smaller than the internal diameter of the syringe barrel, thereby permitting the cylindrical element to move with the plunger shaft within the syringe barrel and thereby permitting further displacement of the plunger shaft.

According to yet another preferred embodiment, the releasable stop or limiting means is provided as a pair of flaps extending from and pivotable with respect to a central portion of the plunger shaft. In a deployed position, the flaps extend outward beyond the internal diameter of the syringe barrel such that the flaps are adapted to engage the syringe barrel flange and prevent movement of the plunger. In a retracted position, the flaps are pivoted about the central portion of the plunger shaft to a position in which the flaps are folded about the central portion of the plunger shaft, thereby permitting the plunger shaft to undergo continued displacement within the syringe barrel.

According to still another preferred embodiment, the releasable stop or limiting means is provided as a lever pivotably attached to the plunger shaft at a generally central location and having an actuating handle that extends upward beyond the plunger push button. A slot is formed in the plunger push button to accommodate radially inward movement of the actuating handle. On an end of the lever opposite the actuating handle is an engaging end of the lever. When the actuating lever resides in the slot in the plunger push button, the lever is in a deployed position in which the engaging end is positioned to engage the syringe barrel flange. When the lever actuating handle is moved radially outward from the slot in the push button, the lever assumes a retracted position in which the engaging end is disposed adjacent the plunger shaft to permit further displacement of the plunger shaft within the syringe barrel.

According to yet another preferred embodiment, the releasable stop or limiting means is provided as a lever extending from the plunger shaft and pivoting about a pivot joint disposed at an end of the lever opposite an actuating handle. The actuating handle, when the lever is in a deployed position, is disposed in a position remote from the plunger push button and a recess or catch formed on the lever engages a complementarily-shaped annular projecting lip formed on the barrel flange. The actuating handle may be moved to a retracted position by application of a radially inward force, causing the handle to move into a slot formed in the posh button and causing the recess or catch to become disengaged from the annular projecting lip, thus permitting continued displacement of the plunger within the syringe barrel.

According to yet another preferred embodiment, the releasable stop or limiting means is provided as a pair of projections extending from the plunger push button. The plunger shaft and push button are permitted to rotate about a longitudinal axis of the plunger shaft relative to the syringe barrel. In a deployed position, the projections are rotated to a position where they engage the syringe barrel flange and therefore prevent movement of the plunger shaft. In a retracted position, the projections are rotated to a position where they clear the syringe barrel flange and therefore permit movement of the plunger within the syringe barrel.

According to yet another preferred embodiment, the releasable stop or limiting means is provided as a breakaway projection extending from the syringe barrel flange or from the plunger shaft and adapted to engage the push button of the syringe plunger in a deployed position. The breakaway projection is moved to its retracted position by separating the breakaway projection from the syringe barrel, preferably at a frangible or weakened portion of the breakaway projection. With the breakaway projection removed, further downward travel of the plunger is permitted within the barrel flange.

According to yet another preferred embodiment, the releasable stop or limiting means is provided as a pair of interfering rings, one formed on the plunger shaft and one formed on an interior surface of the syringe barrel. Upon application of a suitable force to the syringe plunger push button, one or both of the rings deforms from a deployed position to a retracted position such that the plunger shaft ring is permitted to pass the syringe barrel ring and further movement of the plunger within the syringe barrel is permitted.

According to yet another preferred embodiment, the releasable stop or limiting means is provided as a leaf spring formed on the plunger shaft and having a tab or projection for engaging an internal annular ledge formed on the syringe barrel interior. The ledge extends inward to engage the tab or projection as the plunger shaft is displaced within the syringe barrel and provides suitable tactile indication to a user that the plunger displacement has reached a first limit. Upon application of a suitable increased force to the plunger push button by the user, the annular ledge causes the leaf spring to moves from a deployed position to a retracted position in which further movement of the plunger into the syringe barrel is permitted.

According to yet another preferred embodiment, the releasable stop or limiting means is provided as a removable element disposed in a slot formed in the plunger shaft. The removable element is of a generally U-shaped construction and includes a recess formed therein which cooperates with the slot on the plunger shaft. When the removable element is in a deployed position in which it is disposed in the slot on the plunger shaft, movement of the plunger shaft is limited by interference between the removable element and the barrel flange. When the removable element is moved to a retracted position in which it is separated from the plunger shaft, the plunger shaft is permitted to move within the syringe barrel.

According to yet another preferred embodiment, the releasable stop or limiting means is provided as a breakaway cylindrical element disposed around the plunger shaft. The cylindrical element has an outer diameter that is greater than the inner diameter of the syringe barrel such that an engaging end of the cylindrical element engages the syringe barrel flange and an opposite end of the cylindrical element engages the plunger push button. In this deployed position, movement of the plunger relative to the syringe barrel is limited. The cylindrical element is moved to its retracted position by fracturing the cylindrical element, preferably into two halves along one or more weakened or frangible lines or areas. Fracture of the cylindrical element permits removal and further movement of the plunger within the syringe barrel.

According to yet another preferred embodiment, the releasable stop or limiting means is provided as a pair of pivotable finger tabs extending outward from a central portion of the plunger shaft. When the finger tabs are pivoted to a deployed position, stop edges on the finger tabs are adapted to engage the syringe barrel. When the finger tabs are pivoted to a retracted position, the stop edges are disposed in a position where they do not engage the syringe barrel and further movement of the plunger shaft within the syringe barrel is permitted.

According to still another preferred embodiment, the releasable stop or limiting means is provided as an externally threaded ring on the plunger shaft which cooperates with an internally threaded insert on the syringe barrel. Upon downward movement of the plunger shaft within the syringe barrel, the externally threaded ring engages the internally threaded insert and prevents further movement of the plunger shaft. To continue movement of the plunger shaft within the syringe barrel, the plunger shaft must be rotated about a longitudinal axis relative to the syringe barrel, thus causing the externally threaded ring to become threaded into and through the internally threaded insert and permitting further movement of the plunger shaft within the syringe barrel.

Numerous other advantages and features of the present invention will become readily apparent from the following detailed description of the invention, from the claims, and from the accompanying drawings.

### BRIEF DESCRIPTION OF THE DRAWINGS

In the accompanying drawings that form part of the specification, and in which like numerals are employed to designate like parts throughout the same,
FIG. 1A is a partial cross-sectional view of a first embodiment of an exemplary syringe according to the present invention showing the syringe prior to discharge of a diluent or first constituent, with the releasable stop being in the deployed position;
FIG. 1B is a partial cross-sectional view of the embodiment of FIG. 1A showing the displacement of the syringe plunger being limited by the releasable stop;
FIG 1C is a partial cross-sectional view of the embodiment of FIG. 1A showing the releasable stop in the retracted position, permitting further displacement of the diluent or first constituent from the syringe;
FIG. 1D is a cross-sectional view in reference to line 1D-1D of FIG. 1B;
FIGS. 2A-2E are partial cross-sectional views of a syringe system including an embodiment of a syringe similar to that shown in FIG. 1A in an exemplary combination with a second syringe according to another preferred embodiment of the invention and illustrating the operation of the syringe system to mix and deliver a mixture of diluent and drug and to dispense a residual supply of diluent;
FIGS. 3A and 3B are partial cross-sectional views of an exemplary syringe according to another preferred embodiment of the invention showing a releasable stop in deployed and retracted positions, respectively;
FIG. 3C is a cross-sectional view in reference to line 3C-3C in FIG. 3B;
FIGS. 4A and 4B are partial cross-sectional views of an exemplary syringe according to another preferred embodiment of the invention showing a releasable stop in a deployed and retracted positions, respectively;
FIG. 4C is a cross-sectional view in reference to line 4C-4C in FIG. 4B;
FIG. 5A is a perspective view of an exemplary syringe according to another preferred embodiment of the invention showing a releasable stop in a deployed position;
FIG. 5B is a partial section showing the releasable stop in a retracted position;
FIG. 5C is a cross-sectional view of a plunger shaft in accordance with the embodiment of FIG. 5A;
FIG. 5D is a top view in reference to line 5D-5D in FIG. 5B;
FIGS. 6A and 6B are partial cross-sectional views of an exemplary syringe according to another preferred embodiment of the invention showing a releasable stop in deployed and retracted positions, respectively;
FIG. 6C is a cross-sectional view in reference to line 6C-6C in FIG. 6B;
FIGS. 7A is a perspective view and FIG. 7B is a top view of an exemplary syringe according to another preferred embodiment of the invention showing a releasable stop in deployed and retracted positions, respectively;
FIGS. 8A and 8B are partial cross-sectional views of an exemplary syringe according to another preferred embodiment of the invention showing a releasable stop in deployed and retracted positions, respectively;
FIGS. 9A and 9B are partial cross-sectional views of an exemplary syringe according to another preferred embodiment of the invention showing a releasable stop in deployed and retracted positions, respectively;
FIGS. 10A and 10B are partial cross-sectional views of an exemplary syringe according to another preferred embodiment of the invention showing a releasable stop in deployed and retracted positions, respectively;
FIGS. 11A and 11B are partial cross-sectional views of an exemplary syringe according to another preferred embodiment of the invention showing a releasable stop in deployed and retracted positions, respectively;
FIGS. 12A and 12B are partial cross-sectional views of an exemplary syringe according to another preferred embodiment of the invention showing a releasable stop in deployed and retracted positions, respectively;
FIGS. 12C and 12D are cross-sectional views in reference to line12C-12C in FIG. 12A and line 12D-12D in FIG. 12B, respectively;
FIGS. 13A and 13B are partial cross-sectional views of an exemplary syringe according to another preferred embodiment of the invention showing a releasable stop in deployed and retracted positions, respectively;
FIGS. 13C and 13D are cross-sectional views in reference to line13C-13C in FIG. 13A and line 13D-13D in FIG. 13B, respectively;
FIG. 14A is a perspective view and FIG. 14B is a partial sectional view of an exemplary syringe according to another preferred embodiment of the invention showing a releasable stop in deployed and retracted positions, respectively;
FIGS. 15A and 15B are partial cross-sectional views of an exemplary syringe according to another preferred embodiment of the invention showing a releasable stop in deployed and retracted positions, respectively.

### DETAILED DESCRIPTION OF THE PREFERRED EMBODIMENT

While this invention is susceptible of embodiment in many different forms, this specification and the accompanying drawings disclose only some specific forms as examples of the invention. The invention is not intended to be limited to the embodiments so described. The scope of the invention is pointed out in the appended claims.

Figures illustrating the apparatus show some mechanical elements that are known and that will be recognized by one skilled in the art. The detailed descriptions of such elements are not necessary to an understanding of the invention, and accordingly, are herein presented only to the degree necessary to facilitate an understanding of the novel features of the present invention.

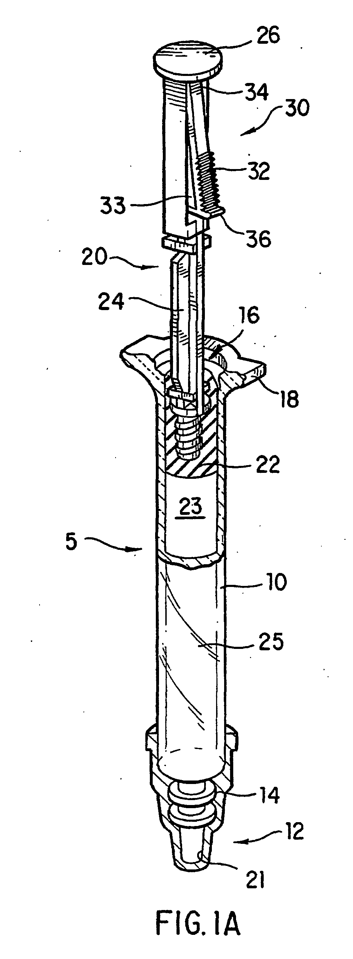

An exemplary syringe according to a first embodiment of the present invention is illustrated in FIGS. 1A through 1D and is designated generally by the reference numeral 5. The syringe 5 includes a generally cylindrical syringe barrel 10 having a discharge end 12 defining a delivery passage 14 therein. Opposite the discharge end 12 is an open end 16 of the syringe barrel 10 and a barrel flange 18 which may typically have a circular or rectangular shape extending from the periphery of the open end 12. A removable closure 21 is provided on the discharge end 12 for sealing the syringe 5 prior to discharge of the diluent 23 contained therein. The barrel 10 is preferably fabricated by injection molding a synthetic polymer, such as a thermoplastic material. Alternatively, the barrel 10 may be made of other suitable materials including glass.

Reciprocally disposed in the open end 16 of the syringe barrel 10 is a plunger 20 for permitting displacement of a piston 22 affixed to an end thereof, typically using a threaded connector. Extending from the piston 22 is a plunger shaft 24 having a push button 26 at an end opposite piston 22. The push button 26 permits a user to apply a displacing force to the plunger 20 and thereby displace diluent 23 from the diluent chamber 25 defined by the syringe barrel 10, discharge end 12 and piston 22.

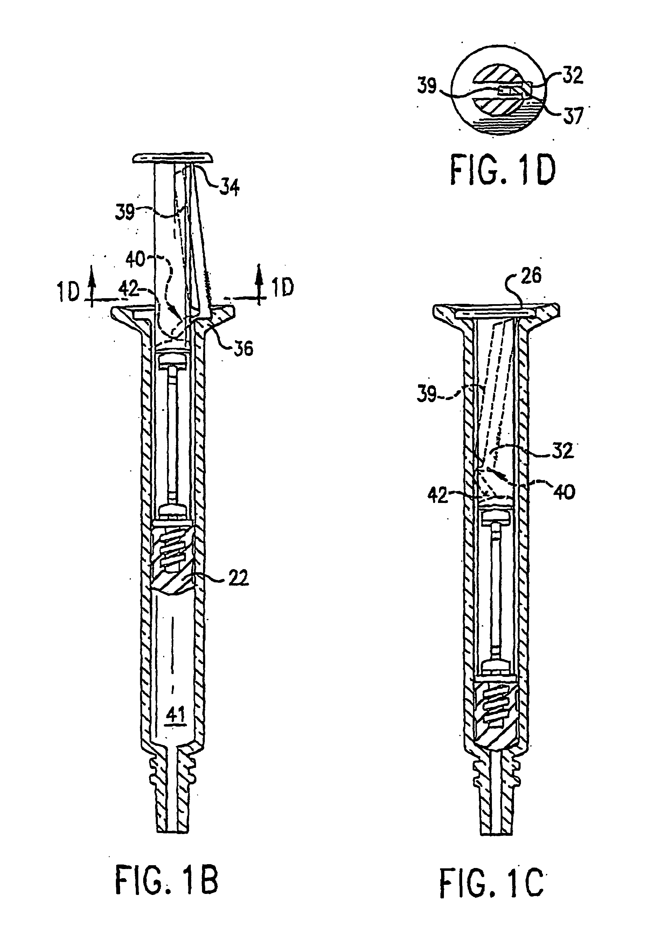

In accordance with the invention, a releasable stop or limiting means 30 is operatively associated with the plunger shaft 24 to selectively limit the displacement of the plunger shaft 24 relative to the syringe barrel 10. Releasable stop 30 comprises a thumb lever 32, which is preferably integrally molded with the plunger shaft 24 and movably disposed within a cavity 33 formed in the plunger shaft 24. The thumb lever 32 includes a pivot end 34 about which the thumb lever 32 pivots relative to the plunger shaft 24 between a deployed position, illustrated in FIGS. 1A, 1B and 1D, and a retracted position, illustrated in FIG. 1C. The thumb lever 32 also includes an engaging end 36 opposite the pivot end 34 for ultimately engaging the barrel flange 18. The thumb lever includes a wider gripping portion 37 and a generally narrow guide portion 39 extending within the cavity 33. The thumb lever 32 also includes a bi-stable, over-center detent member 40 molded therewith for limiting outward movement of the thumb lever 32 relative to the plunger shaft 24 and for providing a biasing force for maintaining the thumb lever 32 in its deployed position and for retaining the thumb lever 32 in the retracted position. The plunger shaft 24, push button 26 and thumb lever 32 are preferably molded as an integral homogenous one-piece construction from polypropylene or acetal. Over-center detent 40 includes a linkage 42 which undergoes a slight compression, and pivots as thumb lever 32 moves from its deployed position to its retracted position. The linkage 42 is constructed to provide a biasing force to the thumb lever 32 in an outwardly direction (toward the deployed position) and provide a biasing force in an opposite inwardly direction (toward the retraced position) once the linkage 42 pivots beyond a center point (as a result of pressure upon the thumb lever 32 from the user) relative to the diluent syringe 10. In this embodiment, the thumb lever 32 is maintained in the retracted position without any aid from the user.

Referring now to FIG. 1C, after a user depresses the thumb lever 32 to the retracted position as shown, the engaging end 36 is moved to an interior position within the cavity 33 formed in the plunger shaft 24. Thus, the plunger 20 is permitted further movement within the syringe barrel 10 and a residual supply 41 (FIG. 1B) of diluent can be displaced from the syringe barrel 10.

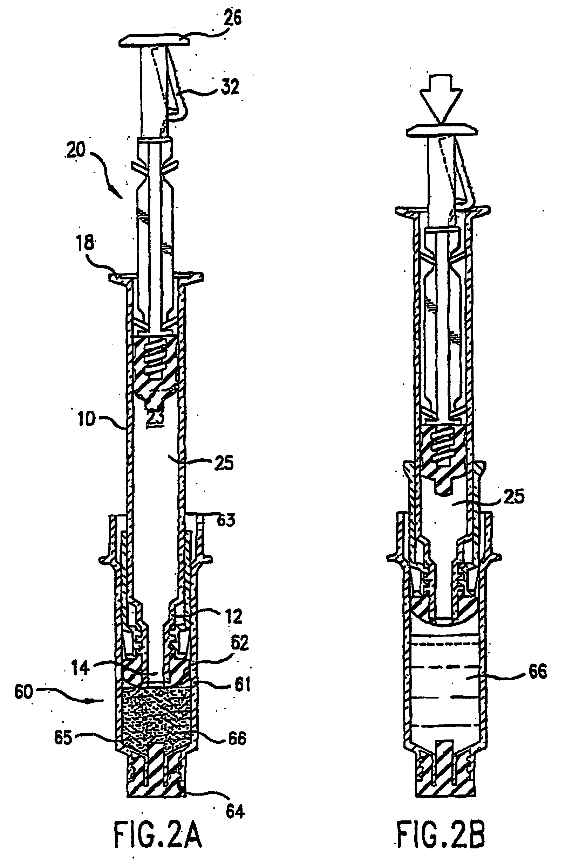

FIGS. 2A - 2E illustrate an exemplary syringe 5 similar to that described relative to FIGS. 1A-1D in combination with a drug syringe, generally referenced by the numeral 60, and having a drug syringe barrel 61 containing a supply of dry drug 65. Drug syringe barrel 61 has an open end 63 opposite a delivery or dispensing end 64. This general combination, exclusive of the features of the present invention, is described in U.S. Patent No. 5,876,372. Referring to FIGS. 2A and 2B, during a mixing stage, a closure, typically a iner cap, is first removed from the diluent syringe 5 thereby permitting egress of the diluent through the discharge passage 14. A back cap (not shown) is also removed from the secondary syringe 60 to permit insertion of the diluent syringe 5 in such a fashion that the discharge end 12 extends through a passage in a stopper element 62 provided on the secondary syringe 60. This ensures a secure fluid connection between the diluent syringe 5 and the mixing chamber 66 defined between the stopper 62 and a delivery end 64 of the drug syringe 60.

Referring to FIG. 2B, the next step in the operation of the device provides for mixing of the diluent 23 and dry drug 45. During this step the plunger 20 of the diluent syringe 5 is depressed thereby displacing a fust amount of diluent from the chamber 25 of the diluent syringe 5 and into the mixing chamber 66 of the secondary syringe 60 where the diluent 23 can be mixed with the dry drug. As diluent 23 is discharged into the mixing chamber 66, the stopper 62 of the drug syringe 60 is displaced (upwards in FIG. 2B) relative to the drug syringe barrel 61. According to the invention, the releasable stop 30 provided on the diluent syringe 5 provides for discharge of a first amount of diluent from the chamber 25 - an amount necessary for proper reconstitution of the dry drug 65 - while a residual supply 41 of diluent remains in the diluent chamber 25.

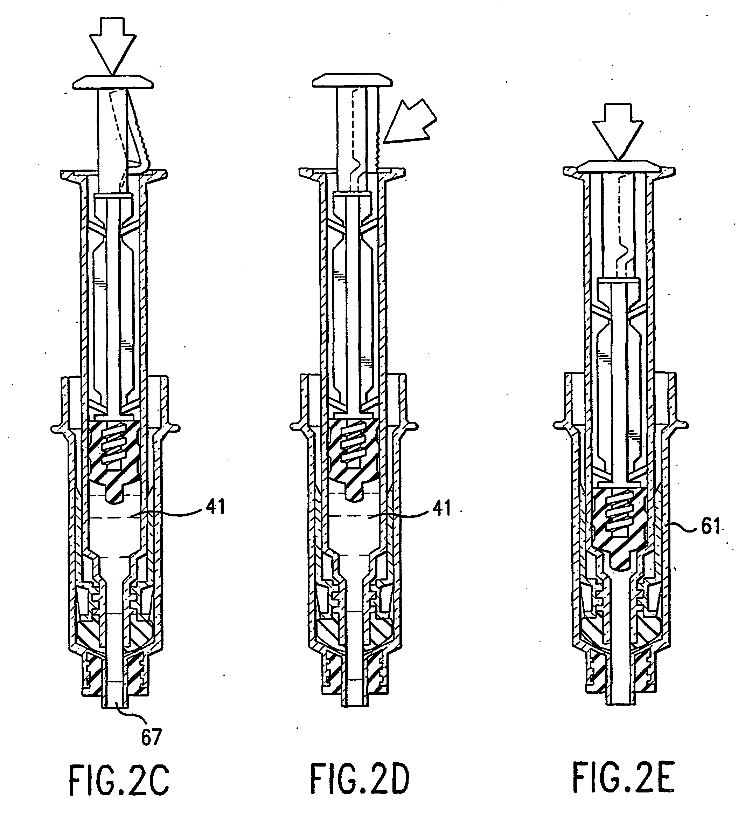

Referring now to FIG. 2C, the mixture of the first amount of diluent 23 and the dry drug 65 is dispensed from the secondary syringe as the user applies a continued downward force on the push button 26, thereby displacing the diluent syringe 5 and stopper 62 of the drug syringe 60 within the drug syringe barrel 61 to displace the now-mixed drug from the mixing chamber 66 and out through the dispensing passage 67 formed in the dispensing end of the secondary syringe. As will be appreciated by those of ordinary skill in the at a residual amount of mixed drug remains in the mixing chamber 66 and within the dispensing passage 67 formed in the dispensing end 64 of the drug syringe 60.

Refening to FIGS. 2D and 2E, the residual supply of mixed drug may be flushed out using the residual supply 41 of diluent that is retained in the diluent syringe 5. To do this, an operator will depress the thumb lever 32 thereby permitting the plunger to undergo a further displacement within the first barrel and permitting the residual supply of diluent 41 to flow into the mixing chamber 66 to flush any residual drug out from the mixing chamber for delivery to the patient. As will be understood by those of ordinary skill in the art, the additional diluent does not create any adverse conditions within the patient and the amount of drug provided in the syringe can be closely controlled to permit a precise amount of drug to be delivered to the patient assuming that the entire amount of drug will be dispensed from the mixing chamber. This is contrast to the prior art where a residual supply of drug would remain in the syringe and therefore the actual amount of drug provided in the syringe during packaging would have to be above the amount required for patient dosage.

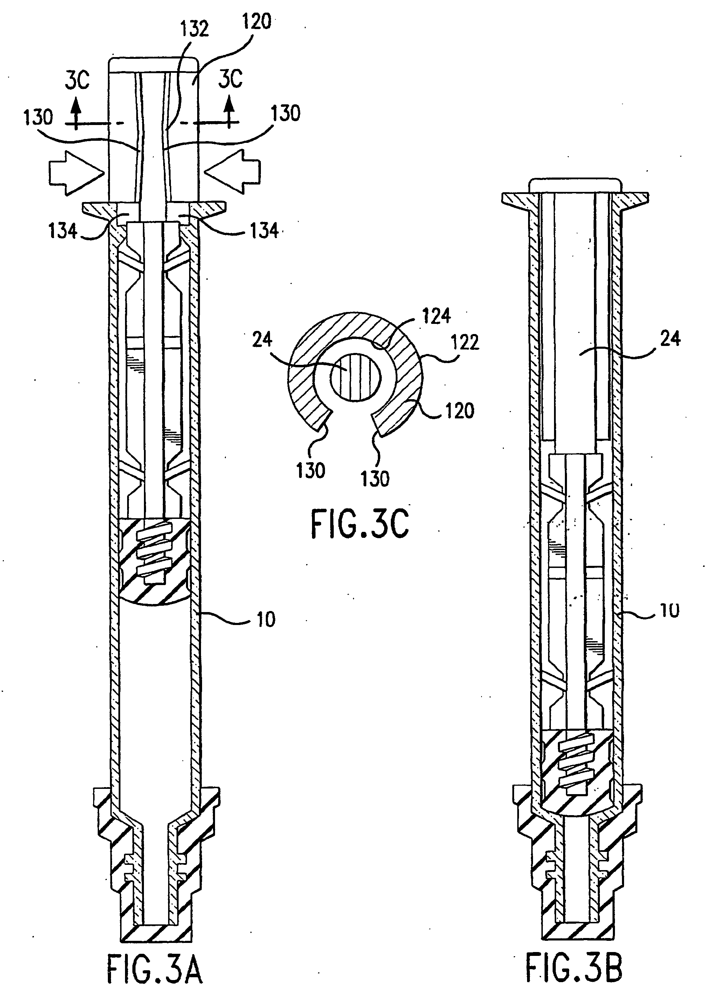

The remaining figures illustrate various other exemplary ways of providing the releasable stop or limiting means on a syringe in accordance with the invention. FIGS. 3A through 3C illustrate an exemplary syringe according to another preferred embodiment of the invention. Here a releasable stop is provided in the form of a compressible cylindrical element 120 which includes an outer surface 122 and an inner surface 124. The compressible cylindrical element 120 includes a slot forming two opposing slot faces 130 which are tapered to provide an apex 132 at a central portion thereof. The compressible element 120 is disposed around the shaft 24 of the plunger 20 and is compressible to a smaller diameter such that an insertion end 134 of the compressible element 120 may be inserted into the inner diameter of the syringe barrel 10. Thus a user may apply lateral force to the sides of the cylindrical element 120 to force the slot faces together and compress an end of the cylindrical element 120, thereby permitting the insertion end 134 to be inserted into the syringe barrel 10 with continued downward force on the plunger button resulting in continued compression of the cylindrical element until it eventually assumes a retracted position and resides entirely within the syringe barrel 10 as shown in FIG. 3B. As will be understood by those of ordinary skill in the art, the compressible cylindrical element provides for a releasable stop which is adapted to move between a deployed position as shown in FIG. 3-A and a retracted position shown in FIG. 3-B. Thus the compressible element interacts with the plunger 24 to provide for displacement of a first amount of diluent or other constituent from the syringe barrel while retaining a residual supply of diluent therein.

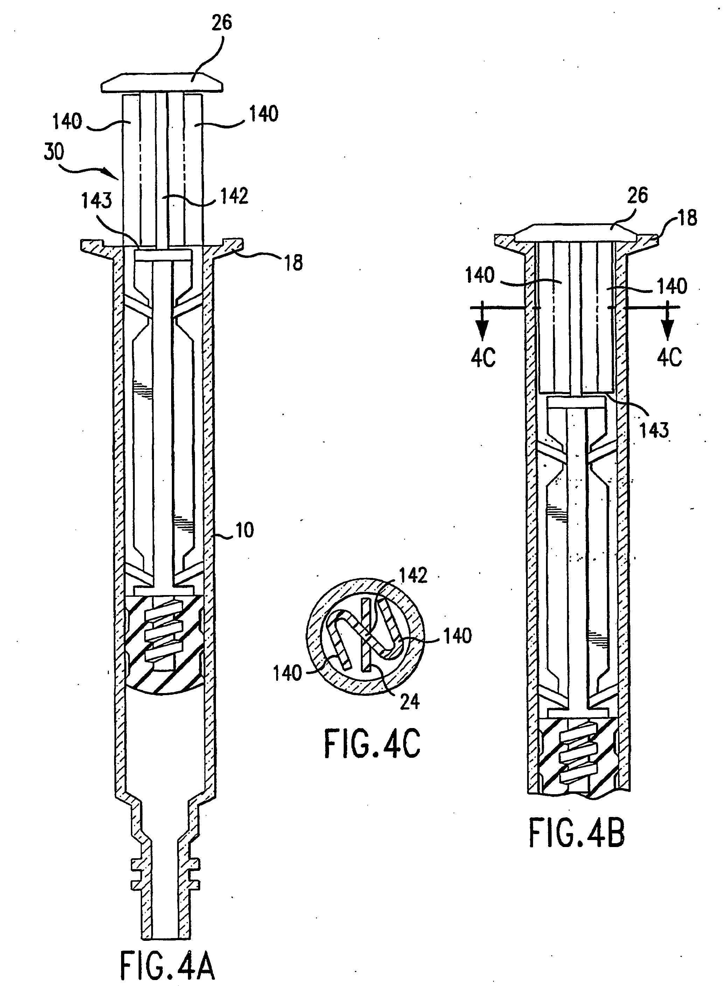

Referring now to FIGS. 4A-4C, there is illustrated another exemplary syringe according to yet another embodiment of the present invention. In this embodiment, the releasable stop 30 or limiting means is provided in the form of a pair ofpivotable flaps 140 depending from a central portion 142 of the plunger shaft 24. The flaps 140 may be molded with a live joint which may comprise a molded plastic area of slight dimension enabling the elements to pivot with respect to the central portion 142 of the plunger shaft. As shown in FIG. 4A, the flaps 140 are in a deployed position and extend outward from the central portion of the plunger shaft where an engaging end 143 of each of the flaps 140 may engage the barrel flange 18. Thus in the deployed position, the flaps 140 limit the displacement of the plunger shaft 24 within the syringe barrel. Referring now to FIGS. 4B and 4C, the releasable stop 30 is shown in a retracted position in which the flaps 140 are pivoted to an inward position in such a fashion that the engaging ends 143 of the flaps 140 no longer engage the barrel flange 18 and the plunger 24 is therefore permitted to be further displaced within the syringe barrel 10.

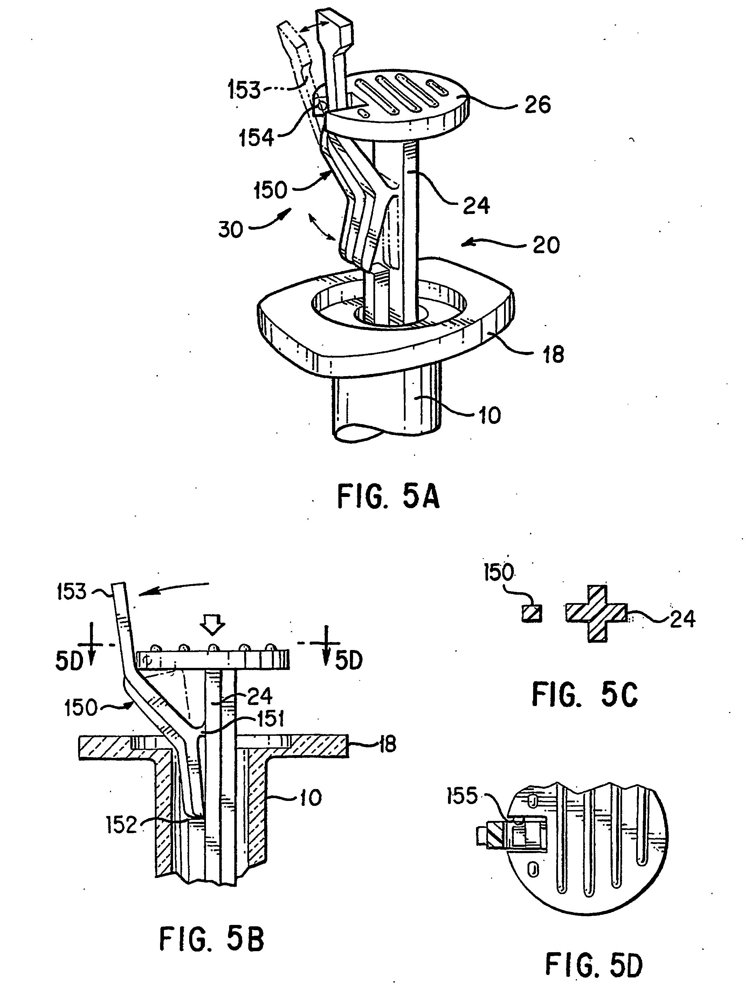

Referring now to FIG. 5A, there is illustrated yet another exemplary syringe in accordance with yet another preferred embodiment of the present invention. In this embodiment, the releasable stop 30 or limiting means is provided as a pivotable lever 150 that is integrally molded with the plunger shaft 24. The lever is provided with a central pivot 151 which may include a living hinge molded of a slight dimension to thereby permit a slight deformation and therefore pivoting of the lever 150. The lever also includes an engaging end 152 for engaging a corresponding portion of the syringe barrel flange 18. As shown in FIG. 5C, the lever extends from a plunger shaft 24 of generally X-shaped cross section. At an end opposite the engaging end 152, the pivotable lever 150 is provided with an actuating handle 153 which extends upward beyond the plunger push button 26. FIG. 5A illustrates the lever 150 in its deployed position in which the stopping surface or engaging surface of the lever 150 is positioned to engage the syringe barrel flange 18. In accordance with a feature of this embodiment, as shown in FIG. 5D, the push button 26 is provided with a recess 154 for receiving the actuator handle 153 and thereby permitting the actuator handle 153 to undergo a radial inward displacement beyond the peripheral extent of the push button 26. Also in accordance with this aspect of the invention, the push button 26 is provided with a pair of retaining protuberances 155 on the recess to retain the actuator handle 153 therein unless overcome by suitable force applied by the user. Thus to move the lever 150 from its deployed position to its retracted position, the user must overcome the resistive forces provided by the protuberances 155 and thereby deform the protuberances or the actuating handle 153 to such a degree that lateral movement of the actuating lever 153 in an outward radial direction relative to the push button 26 is permitted. Such movement therefore results in the lever 150 assuming a retracted position, as shown in FIG. 5B in which the engaging surface is no longer positioned to engage the syringe barrel flange 18 and wherein the plunger shaft is permitted to undergo further displacement downward into the syringe barrel 10. Those of ordinary skill will recognize that the contour of the lever will be provided in such a fashion to provide minimal interference with the syringe barrel 10 as the plunger 20 is displaced downward into the syringe barrel 10.

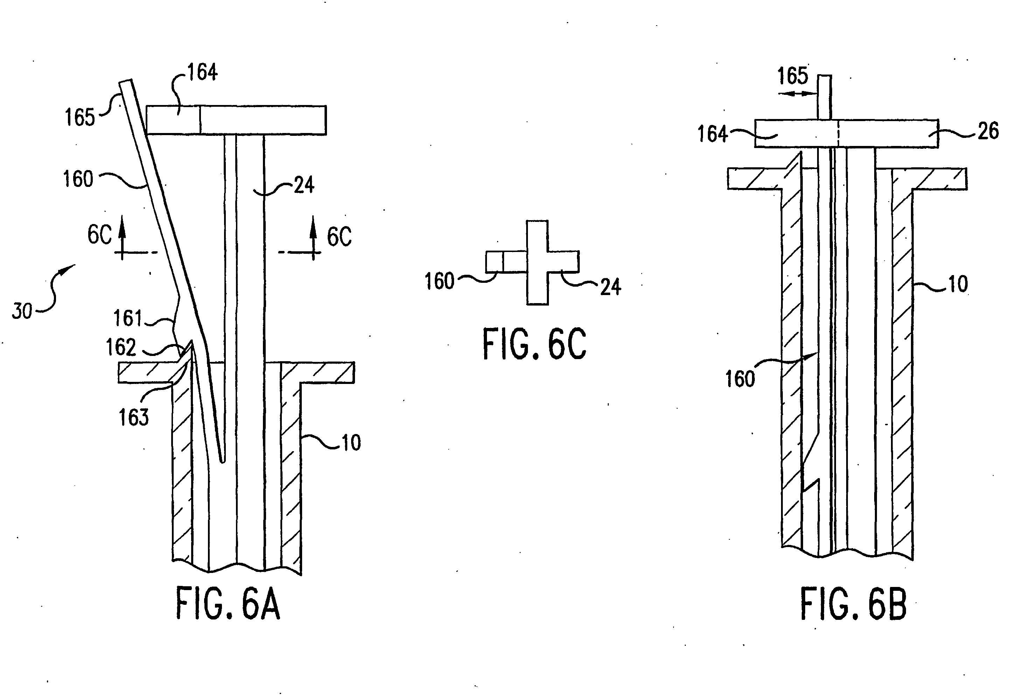

Referring now to FIG. 6A - 6C, there is illustrated another exemplary syringe in accordance with yet another preferred embodiment of the invention. In this embodiment, the releasable stop 30 or limiting means is provided as a flexible lever 160 extending from and formed integrally with the plunger shaft 24. Preferably, the lever 160 is formed with a radially outward bias such that a force is required to displace the lever in a radially inward direction. FIG. 6A illustrates the lever 160 in a deployed position in which a central portion 161 of the lever is provided with a notch or recess 162 which is adapted to engage complementarily-shaped annular lip 163 formed on the syringe barrel 10. As shown in FIG. 6A, the push button 26 of the plunger is provided with a recess 164 similar to the recess 154 described relative to the embodiment shown with respect to FIGS. 5-A through 5-D. Recess 164 permits a further radially inward displacement of an actuator handle 165 of the lever 160 in order to move the lever 160 from the deployed position shown in FIG. 6A to the retracted position shown in FIG. 6B. FIG. 6-C is a cross section taken along lines 6C - 6C of FIG. 6A illustrating that the lever is formed as an integral part of the plunger shaft which has a generally X-shaped cross section. To move the actuator lever 160 from the deployed position to the retracted position, the user applies a radially inward force to the actuator handle 165 thereby displacing the actuator lever 160 to a radially inward position and thereby disengaging the notch or recess 162 of the lever 160 from the annular lip 163 formed on the syringe barrel. It will be noted that a slightly upward force may have to be applied to the plunger shaft 24 in order to remove the recess 162 from the annular lip 163. When the actuator is moved to a radially inward position, the plunger is permitted to undergo further displacement downward and the lever being moved to its retracted position may be further displaced within the syringe barrel.

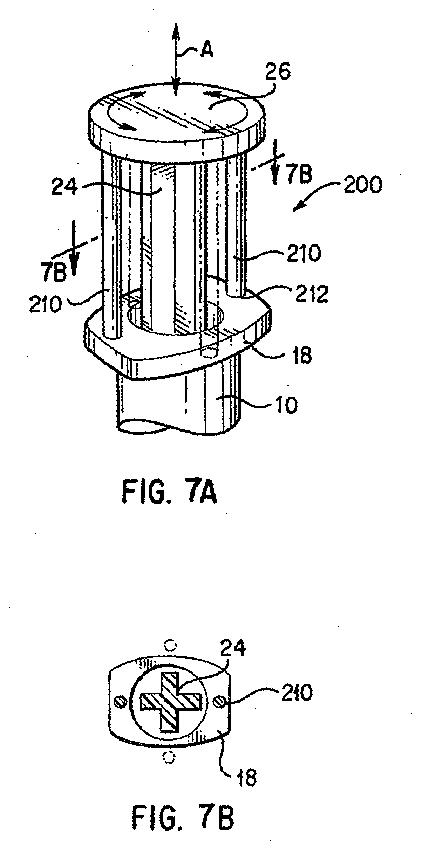

FIGS. 7A and 7B illustrate another exemplary syringe according to yet another preferred embodiment of the present invention. A releasable stop 200 is provided as a pair of projections 210 extending downward from the push button 26 of the plunger 20 to engage the barrel flange 18. The solid lines in FIGS. 7A and 7B illustrate the releasable stop 200 in a deployed position in which push button projections 210 are positioned to engage the barrel flange 18 and thereby prevent further downward movement of the plunger. In accordance with this aspect of the invention, projections 210 are moved to a retracted position, shown in phantom in FIGS. 7A and 7B, by rotation of the plunger 20 and therefore the plunger button 26 about a longitudinal axis (A) relative to the syringe barrel 10. After approximately 90-degrees of rotation in either a clockwise or counterclockwise direction, push button projections 210 are moved to a position in which their respective engaging ends 212 avoid engagement with the barrel flange 18. Thus, the plunger is permitted to undergo further downward displacement upon application of a suitable downward force by a user to the plunger push button. It will be recognized by those of ordinary skill in the art that the barrel flange 18 is preferably formed as an element that is not radially symmetrical, thus permitting selective engagement of the projections 210. However, the barrel flange could be provided as a generally radially symmetrical element, but having holes formed therein for receiving the projections 210 and thereby permitting further downward displacement of the plunger when the projections are rotated to the retracted position.

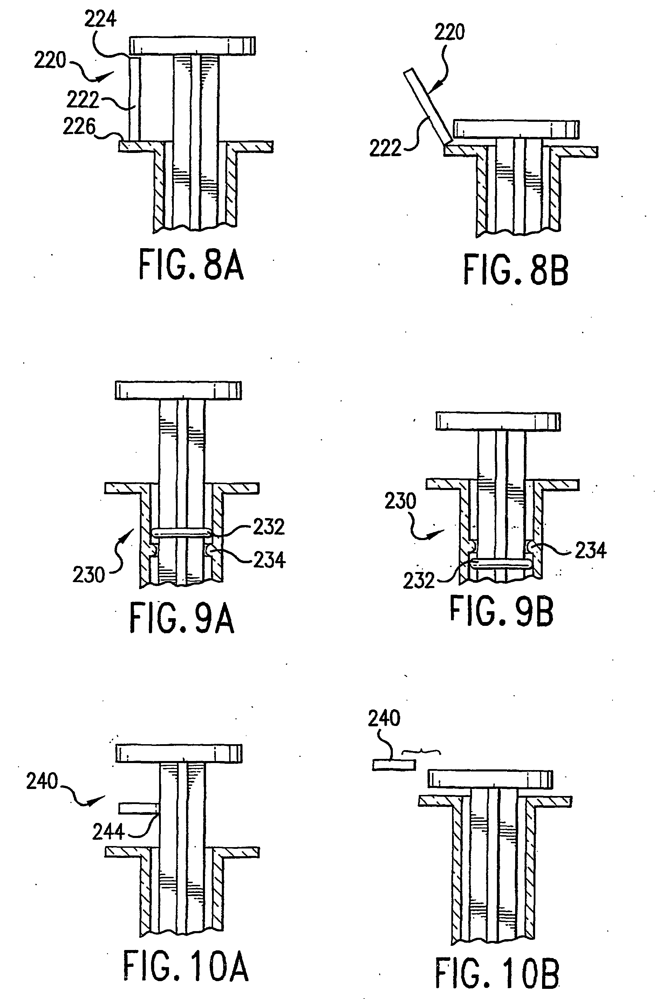

FIGS. 8A and 8B illustrate another exemplary syringe according to yet another preferred embodiment of the present invention. In this embodiment, a releasable stop 220 is provided as a bendable or breakable flange projection 222 extending from the transverse flange of the syringe barrel. Referring to FIG. 8A, in a deployed position, the flange projection 222 extends in a direction generally parallel to the displacement direction of the plunger shaft and is disposed at a radial location relative to the plunger shaft axis such that an engaging end 224 of the projection 222 is positioned to engage the underside of the plunger push button. Flange projection 222 may be formed of the same material as and integrally molded with the syringe barrel. Preferably, flange projection 222 is molded with a weakened or frangible portion 226 enabling relatively quick and easy removal by a user. Flange projection 222 is thus movable to a retracted position shown in FIG. 8B in which flange projection 222 is removed or separated from the transverse flange of the syringe, thereby permitting continued movement of the plunger within the syringe barrel and corresponding displacement of a residual supply of diluent or other constituent from the syringe barrel.

FIGS. 9A and 9B illustrate another exemplary syringe according to yet another preferred embodiment of the present invention. In this embodiment, a releasable stop 230 is provided in the form of two cooperating rings, one formed on the plunger shaft and one formed on the interior of the syringe barrel. A plunger shaft ring 232 may be formed integrally with the plunger shaft and is provided with a radial extent, measured outward from the plunger shaft axis, that is less than the general interior diameter of the syringe barrel. Likewise, the syringe barrel is formed with an interior barrel ring 234 dimensioned to interfere to a controlled degree with the plunger shaft ring 232. Preferably, the dimensions, including the radial extent of the respective rings are selected to require a substantial force before deformation and therefore release of the plunger occurs to prevent inappropriate deformation of the rings. One or both of the plunger shaft ring 232 and/or the interior barrel ring 234 undergoes a deformation and thus moves to a retracted position when a suitable force is applied to the plunger push button to permit additional displacement of the plunger within the syringe barrel and corresponding displacement of the residual supply of diluent or other constituent from the syringe barrel.

FIGS. 10A and 10B illustrate yet another exemplary syringe according to yet another preferred embodiment of the present invention. In this embodiment, a releasable stop 240 is provided as a breakaway projection 242 extending from the plunger shaft. The deployed position of breakaway projection 242 is illustrated in FIG. 10A, where the projection extends radially outward from the plunger shaft and is adapted to engage the transverse flange of the syringe barrel and thereby prevent further displacement of the plunger within the syringe barrel. The retracted position of the breakaway projection 242 is illustrated in FIG. 10B. Here, the breakaway projection 242 is separated from the plunger shaft, which is permitted to undergo additional displacement within the syringe barrel. The breakaway projection 242 is preferably provided with a weakened or frangible portion 244 securing it to the plunger shaft in such a manner that upon application of a suitable force, the breakaway projection 242 may be separated from the plunger shaft. As will be appreciated by those of ordinary skill in the art, separation of the breakaway projection from the plunger shaft preferably occurs by application of a suitable force on the plunger push button and a fracturing or separating force being applied to the breakaway projection 242 by the lateral flange of the syringe barrel.

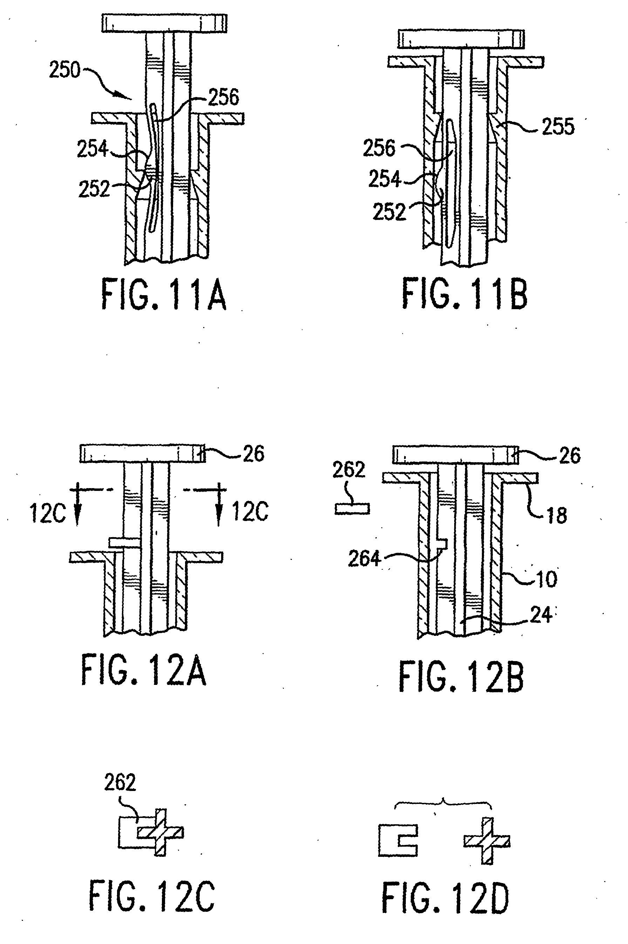

FIGS. 11A and 11B illustrate yet another exemplary syringe according to yet another preferred embodiment of the present invention. In this embodiment, a releasable stop 250 is provided as a leaf spring 252 formed integrally with the plunger shaft and cooperating with an annular ledge 255 formed on the interior of the syringe barrel. Leaf spring 252 preferably has a generally curved shape and molded with the plunger shaft to be biased radially outward. A recess 256 is also formed in the plunger shaft to accommodate the leaf spring and thereby permit radially inward displacement of the leaf spring 252 relative to the plunger shaft. Leaf spring 252 includes a generally round or arcuate projection 254 adapted to engage the annular ledge 254. Annular ledge 255 is dimensioned to provide a relatively large displacement of leaf spring 252 as leaf spring 252 moves from a deployed position, shown in FIG. 11A to a retracted position, during engagement with annular ledge 255.

FIGS. 12A-12D illustrate yet another exemplary syringe according to yet another preferred embodiment of the present invention. According to this embodiment, a releasable stop 260 is provided as a removable element 262 cooperating with a slot 264 formed in the plunger shaft. Preferably, removable element 262 is formed as a generally U-shaped element having a recess 266 formed therein. Recess 266 cooperates with the slot 264 formed in the plunger shaft to retain the removable element 262 in a deployed position shown in FIGS. 12A and 12C, where the removable element 262 is positioned to engage the lateral flange of the syringe barrel. FIGS. 12B and 12D illustrate the removable element 262 in a retracted position, where the removable element 262 is removed from the slot 264 formed in the plunger shaft. It will be recognized by those of ordinary skill that removal of the removable element 262 may occur by simple manual operation by a user of the syringe. With the removable element 262 removed to its retracted position, further displacement of the plunger within the syringe barrel and the corresponding displacement of diluent or constituent may occur.

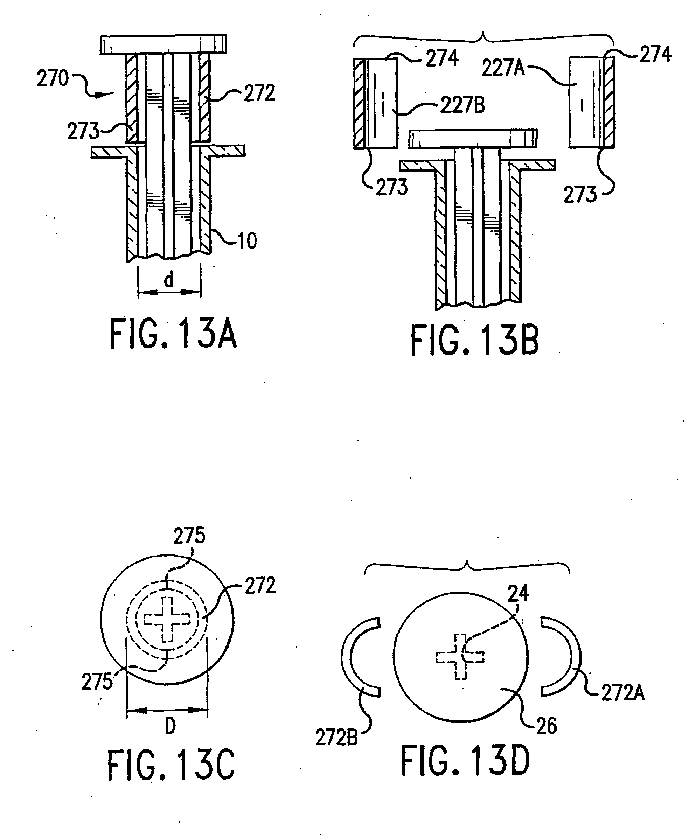

FIGS. 13A-13D illustrate yet another exemplary syringe according to yet another preferred embodiment of the present invention. In this embodiment, a releasable stop 270 or limiting means is provided as a breakaway cylindrical element 272 disposed around the plunger shaft 24 in a deployed position shown in FIGS. 13A and 13C. The breakaway cylindrical element 272 has an outer diameter (D) that is greater than the inner diameter (d) of the syringe barrel 10 such that an engaging end 273 of the breakaway cylindrical element 272 engages the syringe barrel flange 18 and an opposite end 274 of the cylindrical element engages the plunger push button 26. In this deployed position, movement of the plunger relative to the syringe barrel is limited. The breakaway cylindrical element 270 is moved to its retracted position, shown in FIGS. 13B and 13D by fracturing the cylindrical element, preferably into plural portions 272A and 272B along one of more weakened or frangible lines or areas 275. Fracture of the cylindrical element permits removal and further movement of the plunger 20 within the syringe barrel 10.

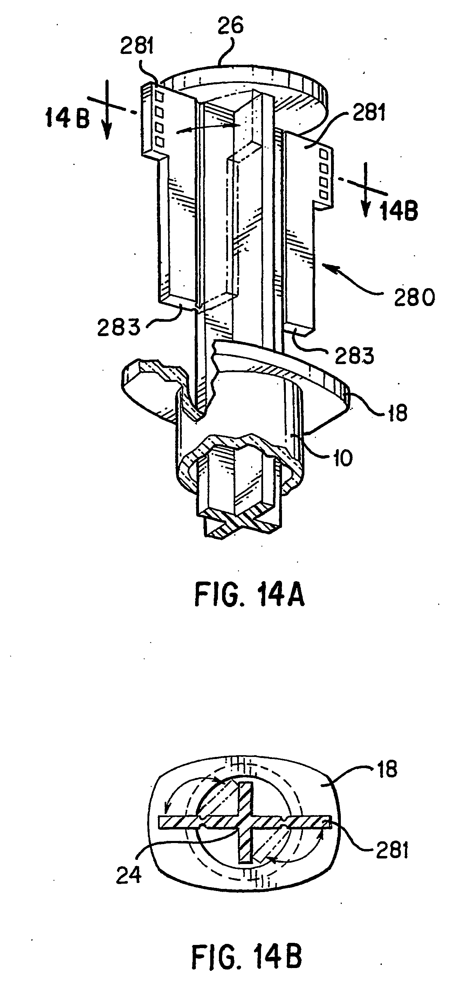

FIGS. 14A and 14B illustrate yet another exemplary syringe according to yet another preferred embodiment of the invention. In this embodiment, a releasable stop 280 is provided as a pair of pivotable finger tabs 281 extending outward from a central portion 282 of the plunger shaft 24. When the finger tabs 281 are pivoted to a deployed position, shown in solid lines, stop edges 283 on the finger tabs are adapted to engage the syringe barrel. When the finger tabs are pivoted to a retracted position, shown in phantom, the stop edges 283 are disposed in a position where they do not engage the syringe barrel flange 18 and further movement of the plunger shaft 24 within the syringe barrel 10 is permitted.

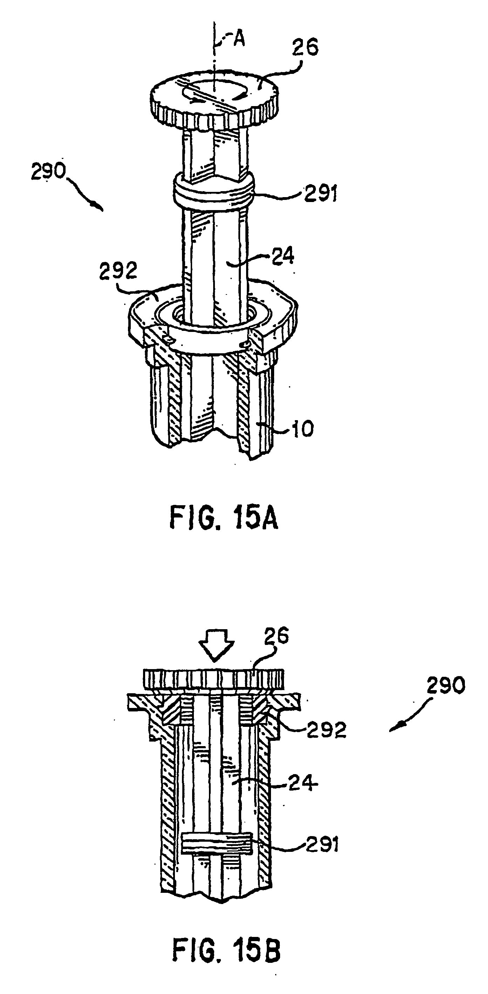

FIGS 15A and 15B illustrate yet another preferred embodiment of the invention. In this embodiment, the releasable stop 290 or limiting means is provided as an externally threaded ring 291 on the plunger shaft 24 which cooperates with an internally threaded insert 292 on the syringe barrel 10. Upon downward movement of the plunger shaft 24 within the syringe barrel 10, the externally threaded ring 291 engages the internally threaded insert 292 and prevents further movement of the plunger shaft 24. To continue movement of the plunger shaft 24 within the syringe barrel 10, the plunger shaft 10 must be rotated about a longitudinal axis (A) relative to the syringe barrel, thus causing the externally threaded ring 291 to become threaded into and through the internally threaded insert 292 and permitting further movement of the plunger shaft within the syringe barrel.

It will be readily apparent from the foregoing detailed description of the invention and from the illustrations thereof that numerous variations and modifications may be effected without departing from the scope of the novel concepts or principles of this invention, the scope of which is defined in the appended claims.

## Claims

1. A prefilled syringe system for storing and later mixing a first constituent (65) and a second constituent (23) and for dispensing the mixed constituents, the syringe system comprising:
a first barrel (61) having an open end (63) and an opposite mixture delivery end (64) defining a mixture delivery passage (67);
a stopper (62) slidably disposed in the first barrel (61), the stopper (62) and the mixture delivery end (64) defining a first chamber (66) for containing the first constituent (65);
a plunger (20) disposed within the first barrel (61);
a releasable stop (30) operatively associated with said plunger (20);
**characterized in that** a second barrel (10) is disposed within the first barrel (61), the second barrel (10) having an open end (16) and an opposite discharge end (12) defining a discharge passage (14) for conveying the second constituent (23) into the first chamber (66) of the first barrel (61);
the plunger (20) being disposed within the second barrel (10), the plunger (20) and discharge end (12) defining a second chamber (25) within the second barrel (10), the plunger (20) being adapted to move within the second barrel (10) and thereby displace the second constituent (23) from the second barrel (10) through the discharge end (12) and into the first chamber (66) of the first barrel (61) to form a mixture of the second constituent (23) and the first constituent (65) in the first chamber (66);
the second barrel (10) cooperating with the stopper (62) such that movement of the second barrel (10) within the first barrel (61) causes the stopper (62) to move and thereby displace the mixture from the first chamber (66) through the mixture delivery passage (67); and
the releasable stop (30) being adapted to move between a deployed position, in which the plunger (20) movement is limited by the releasable stop (30) such that a first supply of the second constituent (23) is displaced from the second barrel (10) to be mixed with the first constituent (65) while a residual supply (41) of the second constituent (23) remains in the second barrel (10) as the mixture is delivered, and a retracted position, in which further movement of the plunger (20) is permitted to displace the residual supply (41) of the second constituent (23) from the second barrel (10).

2. The syringe system according to claim 1 wherein the plunger (20) comprises a piston (22) for sealingly engaging an inside surface of the second barrel (10), an elongate plunger shaft (24) extending from the piston (22) and a push button (26) at an end of the plunger shaft (24) opposite the piston (22) and wherein the releasable stop (30) comprises a deformable projection (222) extending from the barrel (10) and adapted to engage the push button (26).

3. The syringe according to claim 1 wherein the releasable stop (30) comprises a projection (222) extending from the syringe barrel (10).

4. The syringe according to claim 1 wherein the releasable stop (30) comprises an externally threaded ring (291) on the plunger shaft (24) and further comprises an internally threaded insert (292) on the syringe barrel (10), the releasable stop (30) moving from the deployed position to the retracted position by rotation of the externally threaded ring (291) relative to the internally threaded insert (292).

5. The syringe of claim 1 wherein said releasable stop (30) includes a lever (32) adapted to move between said deployed and retracted positions, said lever (32) includes a detent member (40) for maintaining said lever (32) in the deployed position and for retaining said lever (32) in a retracted position.

6. The syringe of claim 5 wherein said detent member (40) includes a linkage (42) which biases said lever (32) in a first outwardly direction toward said deployed position and biases said lever (32) in a second direction opposite said first direction toward said retracted position when said releasable stop (30) moves inwardly beyond a predetermined position.

## Patentansprüche

1. Ein vorgefülltes Spritzensystem zum Lagern und späteren Mischen eines ersten Bestandteils (65) und eines zweiten Bestandteils (23) und zur Abgabe der gemischten Bestandteile, wobei das Spritzensystem folgendes umfasst:
einen ersten Zylinder (61) mit einem offenen Ende (63) und einem entgegengesetzten Mischungs-Abgabe-Ende, das einen Mischungs-Abgabeweg (67) bestimmt;
einen Stopfen (62), der verschiebar im ersten Zylinder (61) angebracht ist, wobei der Stopfen (62) und das Mischungs-AbgabeEnde (64) eine erste Kammer (66) bestimmen, welche den ersten Bestandteil (65) enthält;
einen Plunger (20), der im ersten Zylinder (61) angebracht ist;
eine lösbare Stopvorrichtung (30), die operativ mit dem Plunger (20) verbunden ist;
**dadurch gekennzeichnet, dass** ein zweiter Zylinder (10) in den ersten Zylinder (61) eingesetzt ist, wobei der zweite Zylinder (10) ein offenes Ende (16) und ein entgegengesetztes Abgabeende (12) hat, das einen Abgabeweg (14) zum Befördern des zweiten Bestandteils (23) in die erste Kammer (66) des ersten Zylinders (61) bestimmt;
wobei der Plunger (20) im zweiten Zylinder (10) angeordnet ist, der Plunger (20) und das Abgabeende (12) eine zweite Kammer (25) innerhalb des zweiten Zylinders (10) bestimmen, wobei der Plunger (20) ausgebildet ist, um sich innerhalb des zweiten Zylinders (10) zu bewegen und um **dadurch** den zweiten Bestandteil (23) aus dem zweiten Zylinder (10) durch das Abgabeende (12) und in die erste Kammer (66) des ersten Zylinders (61) zu verdrängen, um in der ersten Kammer (66) eine Mischung aus dem zweiten Bestandteil (23) und dem ersten Bestandteil (65) zu bilden;
wobei der zweite Zylinder (10) so mit dem Stopfen (62) zusammenwirkt, dass die Bewegung des zweiten Zylinders (10) innerhalb des ersten Zylinders (61) den Stopfen (62) veranlasst, sich zu bewegen und **dadurch** die Mischung aus der ersten Kammer (66) durch den Mischungs-Abgabeweg (67) zu verdrängen; und
wobei die lösbare Stopvorrichtung (30) ausgebildet ist, um sich zwischen einer ausgefahrenen Position, in welcher die Bewegung des Plungers (20) durch die lösbare Stopvorrichtung (30) begrenzt wird, so dass eine erste Menge des zweiten Bestandteils (23) aus dem zweiten Zylinder (10) verdrängt wird, um mit dem ersten Bestandteil (65) gemischt zu werden, während eine Restmenge (41) des zweiten Bestandteils (23) im zweiten Zylinder (10) bleibt, während die Mischung abgegeben wird, und einer zurückgezogenen Position zu bewegen, in welcher weitere Bewegung des Plungers (20) gestattet wird, um die Restmenge (41) des zweiten Bestandteils (23) aus dem zweiten Zylinder (10) zu verdrängen.

2. Das Spritzensystem gemäß Anspruch 1, worin der Plunger (20) folgendes umfasst: einen Kolben (22), um abdichtend in eine innere Oberfläche des zweiten Zylinders (10) einzugreifen, eine verlängerte Plunger-Welle (24), die sich von dem Kolben (22) erstreckt, und einen Druckknopf (26) an einem Ende der Plunger-Welle (24) entgegengesetzt zum Kolben (22) und worin die lösbare Stopvorrichtung (30) einen verformbaren Vorsprung (222) umfasst, der sich vom Zylinder (10) erstreckt und der ausgebildet ist, um in den Druckknopf (26) einzugreifen.

3. Die Spritze gemäß Anspruch 1, worin die lösbare Stopvorrichtung (30) einen Vorsprung (222) umfasst, der sich vom Spritzenzylinder (10) erstreckt.

4. Die Spritze gemäß Anspruch 1, worin die lösbare Stopvorrichtung (30) einen Ring (291) mit Außengewinde an der Plunger-Welle (24) umfasst und weiter einen Einsatz (292) mit Innengewinde am Spritzenzylinder (10) umfasst, wobei sich die lösbare Stopvorrichtung (30) durch Rotation des Rings (291) mit Außengewinde relativ zum Einsatz (292) mit Innengewinde von der ausgefahrenen Position zur zurückgezogenen Position bewegt.

5. Die Spritze von Anspruch 1, worin die lösbare Stopvorrichtung (30) einen Hebel (32) einschließt, der ausgebildet ist, um sich zwischen der ausgefahrenen und der zurückgezogenen Position zu bewegen, wobei der Hebel (32) ein Arretierglied (40) zum Halten des Hebels (32) in der ausgefahrenen Position und zum Halten des Hebels (32) in einer zurückgezogenen Position einschließt.

6. Die Spritze von Anspruch 5, worin das Arretierglied (40) ein Verbindungsglied (42) einschließt, welches den Hebel (32) in eine erste Richtung nach außen zur ausgefahrenen Position hin drückt und den Hebel (32) in eine zweite Richtung entgegengesetzt zur ersten Richtung zu der zurückgezogenen Position hin drückt, wenn sich die lösbare Stopvorrichtung (30) über eine vorbestimmte Position hinaus nach innen bewegt.

## Revendications

1. Dispositif de seringue prérempli pour stocker et mélanger ultérieurement un premier composant (65) et un second composant (23) et pour administrer les composants mélangés, le dispositif de seringue comprenant :
un premier corps cylindrique (61) comportant une extrémité ouverte (63) et une extrémité d'administration de mélange opposée (64) définissant un passage d'administration de mélange (67) ;
une butée (62) disposée de manière coulissante dans le premier corps cylindrique (61), la butée (62) et l'extrémité d'administration de mélange (64) définissant une première chambre (66) pour contenir le premier composant (65) ;
un piston plongeur (20) disposé à l'intérieur du premier corps cylindrique (61) ;
une butée amovible (30) associée de manière opérationnelle audit piston plongeur (20) ;
**caractérisé en ce qu'**un second corps cylindrique (10) est disposé à l'intérieur du premier corps cylindrique (61), le second corps cylindrique (10) ayant une extrémité ouverte (16) et une extrémité d'évacuation opposée (12) définissant un passage d'évacuation (14) pour transporter le second composant (23) dans la première chambre (66) du premier corps cylindrique (61) ;
le piston plongeur (20) étant disposé à l'intérieur du second corps cylindrique (10), le piston plongeur (20) et l'extrémité d'évacuation (12) définissant une seconde chambre (25) à l'intérieur du second corps cylindrique (10), le piston plongeur (20) étant adapté pour se déplacer à l'intérieur du second corps cylindrique (10) et ainsi déplacer le second composant (23) à partir du second corps cylindrique (10) en passant par l'extrémité d'évacuation (12) et dans la première chambre (66) du premier corps cylindrique (61) pour former un mélange du second composant (23) et du premier composant (65) dans la première chambre (66) ;
le second corps cylindrique (10) coopérant avec la butée (62) de sorte que le mouvement du second corps cylindrique (10) à l'intérieur du premier corps cylindrique (61) provoque le déplacement de la butée (62) et ainsi le déplacement du mélange à partir de la première chambre (66) par le passage d'administration de mélange (67) ; et
une butée amovible (30) étant adaptée pour se déplacer entre une position déployée, dans laquelle le mouvement du piston plongeur (20) est limité par la butée amovible (30) de sorte qu'une première alimentation du second composant (23) est déplacée à partir du second corps cylindrique (10) pour être mélangée avec le premier composant (65) alors que l'alimentation résiduelle (41) du second composant (23) reste dans le second corps cylindrique (10) lorsque le mélange est administré, et une position rétractée, dans laquelle le mouvement supplémentaire du piston plongeur (20) est autorisé pour déplacer l'alimentation résiduelle (41) du second composant (23) à partir du second corps cylindrique (10).

2. Dispositif de seringue selon la revendication 1, dans lequel le piston plongeur (20) comprend un piston (22) pour mettre en prise de manière étanche une surface interne du second corps cylindrique (10), un arbre (24) de piston plongeur allongé s'étendant à partir du piston (22) et un bouton poussoir (26) situé à une extrémité de l'arbre (24) du piston plongeur opposé au piston (22) et dans lequel la butée amovible (30) comprend une saillie déformable (222) s'étendant à partir du corps cylindrique (10) et adaptée pour mettre en prise le bouton poussoir (26).

3. Seringue selon la revendication 1, dans laquelle la butée amovible (30) comprend une saillie (222) s'étendant à partir du corps cylindrique (10) de la seringue.

4. Seringue selon la revendication 1, dans laquelle la butée amovible (30) comprend un anneau (291) fileté à l'extérieur sur l'arbre (24) du piston plongeur et comprend en outre un insert (292) fileté à l'intérieur sur le corps cylindrique (10) de la seringue, la butée amovible (30) passant de la position déployée à la position rétractée grâce à la rotation de l'anneau (291) fileté à l'extérieur par rapport à l'insert (292) fileté à l'intérieur.

5. Seringue selon la revendication 1, dans laquelle ladite butée amovible (30) comprend un levier (32) adapté pour se déplacer entre lesdites positions déployée et rétractée, ledit levier (32) comprend un élément de détente (40) pour maintenir ledit levier (32) dans la position déployée et pour retenir ledit levier (32) dans une position rétractée.

6. Seringue selon la revendication 5, dans laquelle ledit élément de détente (40) comprend une tringlerie (42) qui sollicite ledit levier (32) dans une première direction vers l'extérieur, vers ladite position déployée et sollicite ledit levier (32) dans une seconde direction opposée à ladite première direction, vers ladite position rétractée lorsque ladite butée amovible (30) se déplace vers l'intérieur au-delà d'une position prédéterminée.
